# EUROPEAN PATENT APPLICATION

(11) **EP 2 777 403 A1**
(43) Date of publication of application: **17.09.2014**
(21) Application number: 13382088.6
(22) Date of filing: 15.03.2013
(51) Int. Cl.: A23L 1/09, A61K 31/70, A61P 3/04, A61P 3/10, A61P 19/08, A61P 25/28

(54) **Use of specific carbohydrate systems during pregnancy for improving bone development and formation and/or for improving cognitive and cns development in offspring**

(71) Applicant: ABBOTT LABORATORIES, Abbott Park, IL 60064-3500 (US)
(72) Inventor: López Pedrosa, José María, 18190 CENES DE LA VEGA (Granada) (ES); Manzano Martín, Manuel Cristóbal, 18151 OGIJARES (Granada) (ES); Martín Martín, María Jesús, 18006 GRANADA (ES); Bueno Vargas, Mª Pilar, 18012 GRANADA (ES); Oliveros Delgado, Elena, 18008 GRANADA (ES); Rueda Cabrera, Ricardo, 18008 GRANADA (ES)
(74) Representative: Ungria López, Javier

(57) **Abstract**

The present disclosure is directed to the administration of specific carbohydrate systems to a pregnant and/or lactating woman for improving one or more characteristics in offspring, for example cognition and/or bone health. The carbohydrate system may include a slow rate of digestion simple carbohydrate, a complex carbohydrate, and a non-absorbent carbohydrate and/or an indigestible carbohydrate.

## Description

### FIELD OF THE DISCLOSURE

The present disclosure is directed to the administration of specific carbohydrate systems to a pregnant woman for improving the health of offspring, including, for example, improved bone development and formation in the offspring and/or reduced incidence of deficiencies in bone health later in life in offspring, and/or enhanced cognitive development and/or CNS development in offspring. More specifically, the present disclosure is directed to the administration of a carbohydrate system comprising a slow rate of digestion simple carbohydrate, a complex carbohydrate, and a non-absorbent carbohydrate and/or an indigestible carbohydrate to a pregnant woman for obtaining one or more of the above-mentioned benefits to the offspring. Optionally, the carbohydrate system may be administered during lactation to improve bone health and/or cognitive and/or CNS development in the offspring. In one aspect, such administration may reduce long term adverse bone health, improve cognitive development and/or improve CNS development and reduce related co-morbidities later in life in the offspring.

### BACKGROUND OF THE DISCLOSURE

The prevalence of obesity, overweight, and glucose intolerance in adolescents and adults has increased rapidly over the past 20 years in the United States and globally and continues to rise. Overweight and obesity are classically defined based on the percentage of body fat or, more recently, the body mass index or BMI. The BMI is defined as the ratio of weight in kilograms divided by the height in meters, squared. As overweight and obesity become more prevalent in all age groups, it is inevitable that the number of women giving birth who are also overweight and/or diabetic will also increase. It is known that overweight and obese women who become pregnant have a greater risk of developing gestational diabetes. Maternal hyperglycemia may lead to infants with increased body size and fat mass and such infants are themselves prone to develop obesity and diabetes later in life, including during adolescence and adulthood. Additionally, recent research has suggested that obese women who themselves have normal glucose tolerance give birth to infants with a higher fat mass than those born to women who are not obese. An increasing body of scientific evidence suggests that infants born to overweight and obese mothers have a greater risk of becoming overweight or obese later in life than infants born to mothers who are not overweight or obese. This predisposition appears to be higher if both parents are affected. Childhood overweight and obesity currently affects millions of children worldwide.

Obesity is associated with a substantial increase in co-morbidities, such insulin resistance, cardiovascular diseases, and type 2 diabetes ("T2D"). Obesity and diabetes, in turn, are both associated with a negative influence on cognitive behavior. For example, rats fed a high-fat diet for 3 months developed obesity and showed a significant impairment in learning and memory tasks. (Winocur G, et al. Neurobiol Aging 26-supp 1 (2005). Humans with T2D suffered impairment in medial temporal lobe function. (Greenwood CE, Winocur G. Neurobiol Aging 26-supp1 (2005):42-45). Children of diabetic mothers are more prone to develop obesity and diabetes than those of healthy women, i.e., the prevalence of diabetes in the offspring of diabetic mothers is increased up to 70% but remains under 10% in the offspring of non-diabetic mothers (Dabalea D. Diabetes Care 30-supp2 (2007):S169-S174). Further, children born to mothers with either diabetes or gestational diabetes performed worse than control on several neurobehavioral tests conducted at school age (Ornoy A, Ratzon N, Greenbaum C, Wolf A, Dulitzky M. J Pediatr Endocrinol Metab 14 (2001):681-689, Veena SR, Krishnaveni GV, Srinivasan K, Kurpad AV, Muthayya S, Hill JC, Kiran KN, Fall CHD. Diabetologia 53(2010):2134-2138). Additionally, a relationship has been reported between carbohydrate (CHO) meals and cognition. A high glycemic index (GI) seems to be related to poorer performances in different tasks. Hence, scores for young rats fed a sucrose diet to induce obesity were significantly worse in Morris water maze and novel object recognition tasks, denoting impaired learning and memory (Jurdak N, Lichtenstein AH, Kanarek RB. Nutr Neurosci 11(2008):48-54, Jurdak N, Kanarek RB. Physiol Behav 96(2009):1-5). On the other hand, low GI food has been proven effective in improving working memory, executive function, and verbal memory when fed to adult humans with T2D. (Papanikolaou Y, Palmer H, Binns MA, Jenkins DJA, Greenwood CE. Diabetologia 49(2006):855-862).

In addition, obesity and diabetes in the mother during pregnancy may impact the health of the offspring later in life in other aspects, such as bone health and formation. For example, the onset of diabetes in adolescence may result in a decreased peak bone mass, which is an established determinant of bone strength. It has been reported that higher bone mass at skeletal maturity results in a lower risk of development of osteoporosis later in life and subsequently lower fracture risk (Eastell and Lambert 2002).

It would therefore be desirable to provide nutritional compositions and methods that could improve the health of the offspring and prevent or reduce the incidence or risk of multiple diseases or conditions, such as bone health and/or cognition (including CNS development). It would be further beneficial if such methods could be utilized early in development to program the child against such diseases and conditions. The present disclosure addresses one or more of these needs.

### SUMMARY OF THE DISCLOSURE

While prior studies have reported a beneficial effect of low GI meals by improving mood, learning abilities and word recall as compared to high GI meals, such data relates to school age children (Micha R, Rogers PJ, Nelson M. Br J Nutr 106(2011):1552-1561) and adults (Nilsson A, Radeborg K, Björck I. Eur J Clin Nutr 63(2009)113-120). It has now surprisingly been discovered that an improvement in glycemia and insulinemia in a woman during gestational and lactating periods can be obtained by administering to the woman a nutritional composition including a carbohydrate system that includes a slow rate of digestion simple carbohydrate, a complex carbohydrate, a nonabsorbent carbohydrate, and/or an indigestible oligosaccharide, and that such improvement can result in the improvement of one or more characteristics of the offspring via a cross-generational mechanism. For example, it has been discovered by Applicant that, by administering a nutritional formula containing a carbohydrate system as disclosed herein to a mother during gestation, offspring have a significant increase in bone mass, a key determinant for developing an individual's ability to exercise and prevent bone fragility later in life, and improved cognitive function.

The present disclosure is therefore directed to the administration of specific carbohydrate systems, which may be part of a nutritional composition, to a pregnant woman for improving a characteristic of offspring. Such characteristic may include, for example, bone health and/or cognitive development. Specifically, the present disclosure is directed to the administration of a carbohydrate system comprising a slow rate of digestion simple carbohydrate, such as isomaltulose, and a complex carbohydrate, such as a maltodextrin, in combination with a non-absorbent carbohydrate, such as an insoluble dietary fiber, and/or an indigestible carbohydrate, such as fructooligosaccharides, to a pregnant woman, and optionally to the woman during lactation, to improve the health of the offspring, in particular, with regard to improved bone health and/or improved cognitive performance.

Thus, in one embodiment, the present disclosure is directed to a method of improving cognitive development in an offspring. The method may comprise administering to a pregnant woman a nutritional composition comprising a carbohydrate system. The carbohydrate system may comprise a slow rate of digestion simple carbohydrate, a complex carbohydrate, a nonabsorbent carbohydrate, and an indigestible oligosaccharide. In one aspect, the pregnant woman is overweight, obese, and/or diabetic. In one aspect, the administration of the composition may occur during lactation.

In another embodiment, the present disclosure is directed to a method of improving bone health in an offspring. The method may comprise administering to a pregnant woman a nutritional composition comprising a carbohydrate system. The carbohydrate system may comprise a slow rate of digestion simple carbohydrate, a complex carbohydrate, a nonabsorbent carbohydrate, and an indigestible oligosaccharide. In one aspect, the pregnant woman is overweight, obese, and/or diabetic. In one aspect, the administration of the composition may occur during lactation.

In addition to the methods of improving a characteristic in an offspring as described in this patent application, the disclosure further relates to nutritional compositions as described herein as applied in said methods. That is, the disclosure further relates to a nutritional composition comprising a carbohydrate system, the carbohydrate system comprising a slow rate of digestion simple carbohydrate, a complex carbohydrate, a nonabsorbent carbohydrate, and/or an indigestible oligosaccharide, for improving a characteristic in an offspring selected from bone health or cognition, by administering the composition to a woman during the gestational and/or lactation period of said offspring. This definition of the composition also covers all the embodiments described and/or claimed in the present text for the method of improving a characteristic in an offspring.

In another embodiment, the present disclosure is directed to a nutritional composition comprising a carbohydrate system. The carbohydrate system comprises isomaltulose, maltodextrin having a DE of 9 to 16, fructooligosaccharides, and an insoluble dietary fiber.

### BRIEF DESCRIPTION OF THE FIGURES

**Figure 1** is a schematic representation of the experimental study of the cognition Example.
**Figure 2** depicts latency to platform(s) during the training blocks (each blocks summarizes data from two consecutive days). Data are expressed as mean ± SEM (n=8).
**Figure 3** depicts the ratio between times spent in the target and the opposite quadrants. Data are expressed as mean ± SEM (n=8).
**Figure 4** depicts the percentage of time spent observing the familiar and the novel object during the novel object recognition task. Data are mean ± SEM (n=8).
**Figure 5** is a schematic representation of the experimental study of the bone health Example.
**Figures 6a-6d** depict adolescence bone architecture in control animals, high fat/high GI diet (HF High GI) and high fat/low GI diet (HF low GI), including male femur bone volume fraction (BV/TV; Fig. 6a), male femur bone surface/bone volume (BS/BV; Fig. 6b), male femur trabecular thickness (Tb.Th; Fig. 6c), and male femur trabecular thickness structure model index (Tb.SMI; Fig 6d).

### DETAILED DESCRIPTION OF THE DISCLOSURE

The nutritional compositions and methods of the present disclosure utilize a specific carbohydrate system. The carbohydrate system, which may include all of the carbohydrate components of the nutritional composition, or only a part of the overall carbohydrate component of the nutritional composition, includes the combination of a slow rate of digestion simple carbohydrate, a complex carbohydrate, a nonabsorbent carbohydrate, and an indigestible oligosaccharide. The nutritional compositions including the carbohydrate systems are administered to a pregnant woman, or a pregnant and then lactating woman, to not only improve glycemia and insulinemia in the woman during gestational and lactating periods, but also to improve one or more characteristics in the offspring later in life. The woman receiving the nutritional composition including the carbohydrate system may, in some cases, be an obese woman who may be diabetic or have gestational diabetes mellitus.

The methods of the present disclose using the carbohydrate systems as described herein may provide an easy, convenient, and effective means for improving the health of the pregnant or pregnant and then lactating mother and for improving the long term health of the offspring by improving one or more characteristics of the offspring and reducing the potential for long term adverse health effects in the offspring later in life. As diseases and conditions such as obesity, overweight, diabetes, and glucose intolerance continue to effect women during pregnancy and lactation, , it becomes increasingly important to develop methods of influencing the programming of offspring during the gestational and/or lactation period so as to reduce the risk and/or incidence of associated diseases and conditions at birth or later in life. The methods of the present disclosure allow offspring to be programmed early in life against long term adverse health effects later in life, including decreased bone health or decreased cognitive function as well as related co-morbidities.

These and other optional features of the nutritional compositions and methods of the present disclosure, as well as some of the many other optional variations and additions, are described in detail hereafter.

The terms "retort" and "retort sterilized" are used interchangeably herein, and unless otherwise specified, refer to the common practice of filling a container, most typically a metal can or other similar package, with a nutritional liquid and then subjecting the liquid-filled package to the necessary heat sterilization step, to form a retort sterilized nutritional liquid product.

The terms "aseptic" and "aseptic sterilized" are used interchangeably herein, and unless otherwise specified, refer to the manufacture of a packaged product without reliance upon the above-described retort packaging step, wherein the nutritional liquid and package are sterilized separately prior to filling, and then are combined under sterilized or aseptic processing conditions to form a sterilized, aseptically packaged, nutritional liquid product.

The terms "nutritional formula" or "nutritional product" or "nutritional composition," as used herein, are used interchangeably and, unless otherwise specified, refer to nutritional liquids, nutritional solids, nutritional semi-liquids, nutritional semi-solids, nutritional powders, nutritional supplements, and any other nutritional food product as known in the art. The nutritional powders may be reconstituted to form a nutritional liquid, all of which comprise one or more of fat, protein and carbohydrate, and are suitable for oral consumption by a human.

The term "nutritional liquid," as used herein, unless otherwise specified, refers to nutritional products in ready-to-drink liquid form, concentrated form, and nutritional liquids made by reconstituting the nutritional powders described herein prior to use.

The term "nutritional powder," as used herein, unless otherwise specified, refers to nutritional products in flowable or scoopable form that can be reconstituted with water or another aqueous liquid prior to consumption and includes both spray dried and drymixed/dryblended powders.

The term "nutritional semi-solid," as used herein, unless otherwise specified, refers to nutritional products that are intermediate in properties, such as rigidity, between solids and liquids. Some semi-solids examples include puddings, gelatins, and doughs.

The term "nutritional semi-liquid," as used herein, unless otherwise specified, refers to nutritional products that are intermediate in properties, such as flow properties, between liquids and solids. Some semi-liquids examples include thick shakes and liquid gels.

The term "later in life," as used herein, refers to the period of life from weaning through elderly, including childhood, adolescence and adulthood.

All percentages, parts and ratios as used herein, are by weight of the total composition, unless otherwise specified. All such weights, as they pertain to listed ingredients, are based on the active level and, therefore, do not include solvents or by-products that may be included in commercially available materials, unless otherwise specified.

Numerical ranges as used herein are intended to include every number and subset of numbers within that range, whether specifically disclosed or not. Further, these numerical ranges should be construed as providing support for a claim directed to any number or subset of numbers in that range. For example, a disclosure of from 1 to 10 should be construed as supporting a range of from 2 to 8, from 3 to 7, from 5 to 6, from 1 to 9, from 3.6 to 4.6, from 3.5 to 9.9, and so forth.

All references to singular characteristics or limitations of the present disclosure shall include the corresponding plural characteristic or limitation, and vice versa, unless otherwise specified or clearly implied to the contrary by the context in which the reference is made.

All combinations of method or process steps as used herein can be performed in any order, unless otherwise specified or clearly implied to the contrary by the context in which the referenced combination is made.

The various embodiments of the nutritional compositions of the present disclosure may also be substantially free of any optional or selected ingredient or feature described herein, provided that the remaining nutritional compositions still contain all of the required ingredients or features as described herein. In this context, and unless otherwise specified, the term "substantially free" means that the selected nutritional compositions contain less than a functional amount of the optional ingredient, typically less than 1%, including less than 0.5%, including less than 0.1%, and also including zero percent, by weight of such optional or selected ingredient.

The nutritional compositions and methods of the present disclosure may comprise, consist of, or consist essentially of the essential elements of the products and methods as described herein, as well as any additional or optional element described herein or otherwise useful in nutritional compositions.

### Product Form

The nutritional compositions as described herein for use in the methods of the present disclosure as noted below comprise a carbohydrate system that includes a slow rate of digestion simple carbohydrate, a complex carbohydrate, a nonabsorbent carbohydrate, and an indigestible oligosaccharide. The actual product form of the nutritional composition administered to the pregnant or lactating woman is not critical so long as the carbohydrate system is as described herein. As such, the product forms described herein should be viewed as exemplary and not limiting in any manner as other product forms not listed herein are within the scope of the present disclosure.

The nutritional compositions of the present disclosure may be formulated and administered in any known or otherwise suitable oral product form. Any nutritional solid, semi-solid, liquid, semi-liquid, or powder form, including combinations or variations thereof, are suitable for use herein, provided that such forms allow for safe and effective oral delivery to the individual of the carbohydrate system as described herein. In one specific embodiment, the nutritional composition is in the form of a bar, such as a nutritional bar, weight loss bar, or meal replacement bar.

The exact form of the nutritional composition of the present disclosure is not critical, although it is desirably formulated as dietary product forms, which are defined herein as those embodiments comprising the carbohydrate system as described herein in a product form that also contains at least one of fat and protein, and optionally, additional carbohydrates. The compositions may be formulated with sufficient kinds and amounts of nutrients to provide a sole, primary, or supplemental source of nutrition, or to provide a specialized nutritional product for use in pregnant and/or lactating women afflicted with specific diseases or conditions or with a targeted nutritional benefit.

### Nutritional Liquids

Nutritional liquids include both concentrated and ready-to-feed nutritional liquids. These nutritional liquids are most typically formulated as suspensions, emulsions or clear or substantially clear liquids.

Nutritional emulsions suitable for use may be aqueous emulsions comprising proteins, fats, and carbohydrates. These emulsions are generally flowable or drinkable liquids at from about 1°C to about 25°C and are typically in the form of oil-in-water, water-in-oil, or complex aqueous emulsions, although such emulsions are most typically in the form of oil-in-water emulsions having a continuous aqueous phase and a discontinuous oil phase.

The nutritional liquids may be and typically are shelf stable. The nutritional liquids typically contain up to about 95% by weight of water, including from about 50% to about 95%, also including from about 60% to about 90%, and also including from about 70% to about 85%, of water by weight of the nutritional liquid. The nutritional liquids may have a variety of product densities, but most typically have a density greater than about 1.03 g/mL, including greater than about 1.04 g/mL, including greater than about 1.055 g/mL, including from about 1.06 g/mL to about 1.12 g/mL, and also including from about 1.085 g/mL to about 1.10 g/mL.

The nutritional liquid may have a pH ranging from about 3.5 to about 8, but are most advantageously in a range of from about 4.5 to about 7.5, including from about 5.5 to about 7.3, including from about 6.2 to about 7.2.

Although the serving size for the nutritional liquid can vary depending upon a number of variables, a typical serving size is generally at least about 2 mL, or even at least about 5 mL, or even at least about 10 mL, or even at least about 25 mL, including ranges from about 2 mL to about 300 mL, including from about 100 mL to about 300 mL, from about 4 mL to about 250 mL, from about 150 mL to about 250 mL, from about 10 mL to about 240 mL, and from about 190 mL to about 240 mL.

### Nutritional Powders

The nutritional powders are in the form of flowable or substantially flowable particulate compositions, or at least particulate compositions. Particularly suitable nutritional powder forms include spray dried, agglomerated or dryblended powder compositions, or combinations thereof, or powders prepared by other suitable methods. The compositions can easily be scooped and measured with a spoon or similar other device, wherein the compositions can easily be reconstituted with a suitable aqueous liquid, typically water, to form a nutritional liquid for immediate oral or enteral use. In this context, "immediate" use generally means within about 48 hours, most typically within about 24 hours, preferably right after or within 20 minutes of reconstitution.

### Carbohydrate System

The methods of the present invention utilize a nutritional composition that includes a carbohydrate system as described herein. The carbohydrate system may include all of the carbohydrate components present in the nutritional composition such that the nutritional composition does not contain any other carbohydrates components, or may include only a portion of the carbohydrate components present in the nutritional composition; that is, in some embodiments there are additional carbohydrate components present in the nutritional composition in addition to the carbohydrate system as described herein such as, for example, lactose.

The carbohydrate systems as described herein may comprise specific combinations of carbohydrates provide a low glycemic load, for example, less than about 55, or less than about 50, or less than about 45, or less than about 40, or less than about 35, or less than about 30, or less than about 28, or about 27. As used herein, the experimental glycemic load is determined experimentally by feeding human test subjects 50 g of available CHO comprising 5.5% lactose + 8.8% maltodextrin DE 9-16 +75% Isomaltulose + 10.7% Fibersol 2E after an overnight fast, expressed as a percentage of the response after 50 g anhydrous glucose was taken by the same subject. This procedure is described in Wolever et al, Measuring the Glycemic Index of Foods: Interlaboratory Study, Am J Clin Nutr. 2008 Jan;87(1):247S-257S. This is in contrast to the "daily glycemic load" or "DLG" of the experimental rodent diet, in which DGL is determined theoretically considering the amount of each individual carbohydrate contained in the CHO mixture, the glycemic index of the CHO mixture and the total dietary intake.

The carbohydrate systems of the present disclosure include a simple carbohydrate that has a slow rate of digestion. Simple carbohydrates include those carbohydrates that are comprised of monosaccharide sugars or disaccharide sugars. Carbohydrates that have a slow rate of digestion are those carbohydrates that are low glycemic and low insulinemic and are carbohydrates that generally provide a gradual, relatively low rise in blood glucose over time. Suitable simple carbohydrates that have a slow rate of digestion that are suitable for use in the carbohydrate system include isomaltulose, sucromalt, and combinations thereof. Sucromalt may be made from the enzymatic conversion of sucrose and maltose into a fructose and oligosaccharide liquid syrup. The oligosaccharide is comprised of glucoses linked together by alternating 1,3 and 1,6 linkages.

The simple carbohydrate that has a slow rate of digestion may be present in the carbohydrate system in an amount of from about 40% to about 80% by weight, including from about 40% to about 75% by weight, including from about 40% to about 70% by weight, including from about 45% to about 70% by weight, including from about 50% to about 70% by weight, including from about 55% to about 70% by weight, including from about 60% to about 70% by weight, including from about 65% to about 70% by weight. In some specific embodiments, the simple carbohydrate that has a slow rate of digestion may be present in the carbohydrate system in an amount of about 65% by weight, or even about 66% by weight, or even about 67% by weight, or even about 68% by weight, or even about 69% by weight, or even about 70% by weight.

In addition to the simple carbohydrate that has a slow rate of digestion, the carbohydrate system includes a complex carbohydrate. Complex carbohydrates include those carbohydrates that are chains of three or more single sugar molecules linked together. Suitable complex carbohydrates for use in the carbohydrate system include, for example, maltodextrins. In some particularly desirable embodiments, the maltodextrins may have a Dextrose Equivalent of from about 5 to about 25, or in other aspects, from about 9 to about 16. Other suitable complex carbohydrates in some embodiments include other sources of starches such as, for example, corn starch, rice starch, wheat starch, and the like.

The complex carbohydrate may be present in the carbohydrate system in an amount of from about 1% to about 15% by weight, including from about 2% to about 12% by weight, including from about 2% to about 10% by weight, including from about 3% to about 10% by weight, including from about 4% to about 10% by weight, including from about 5% to about 10% by weight, including from about 6% to about 10% by weight, including from about 7% to about 10% by weight, and including from about 8% to about 10% by weight. In some particularly desirable embodiments, the complex carbohydrate is present in the carbohydrate system in an amount of about 8% by weight, including about 9% by weight, including about 10% by weight.

In addition to the simple carbohydrate that has a slow rate of digestion and the complex carbohydrate, the carbohydrate systems as described herein additionally include at least one of: (1) a nonabsorbent carbohydrate; and (2) an indigestible oligosaccharide. In some embodiments of the present disclosure, the carbohydrate system will comprise, consist essentially of, or consist of a simple carbohydrate that has a slow rate of digestion, a complex carbohydrate, and a nonabsorbent carbohydrate. In other embodiments of the present disclosure, the carbohydrate system will comprise, consist essentially of, or consist of a simple carbohydrate that has a slow rate of digestion, a complex carbohydrate, and an indigestible oligosaccharide. In still other embodiments of the present disclosure, the carbohydrate system will comprise, consist essentially of, or consist of a simple carbohydrate that has a slow rate of digestion, a complex carbohydrate, a nonabsorbent carbohydrate, and/or an indigestible carbohydrate. In some embodiments, as noted above, one or more additional carbohydrates, such as lactose, may be present in addition to the carbohydrate system.

In some embodiments, the carbohydrate system includes a nonabsorbent carbohydrate. Nonabsorbent carbohydrates include fibers and other non-absorbable starches that are not substantially absorbed in the upper intestinal tract so that they pass through to the colon where bacteria ferment them into fatty acids that can be absorbed. These fatty acids may act to heal the lining of the colon. Suitable nonabsorbent carbohydrates include inulin, and insoluble dietary fibers, including Fibersol® fibers, including Fibersol® 2E, which is a digestion resistant maltodextrin, Nutriose®, amylose, or other insoluble fibers, and combinations thereof.

The nonabsorbent carbohydrate may be present in the carbohydrate system in an amount of from about 5% to about 25% by weight, including from about 5% to about 20% by weight, including from about 5% to about 19% by weight, including from about 5% to about 18% by weight, including from about 5% to about 17% by weight, including from about 5% to about 16% by weight, including from about 7.0% to about 16% by weight, including from about 7.0% to about 15.5% by weight. In some embodiments, the nonabsorbent carbohydrate is present in the carbohydrate system in an amount of about 7.0% by weight. In another embodiment, the nonabsorbent carbohydrate is present in the carbohydrate system in an amount of about 15.5% by weight.

In some embodiments, the carbohydrate system includes an indigestible carbohydrate. Indigestible carbohydrates are carbohydrates, including some fibers, that travel through the colon undigested so as to promote digestion and a healthy bowel. Suitable indigestible carbohydrates include fructooligosaccharides, galactooligosaccharides, trans-galactooligosaccharides, xylooligosaccharides, and combinations thereof.

The indigestible carbohydrate may be present in the carbohydrate system in an amount of from about 1.0% to about 18% by weight, including from about 2% to about 17% by weight, including from about 2% to about 15% by weight, including from about 3% to about 15% by weight, including from about 3% to about 14% by weight, including from about 3% to about 13% by weight, including from about 3% to about 12% by weight. In one particularly desirable embodiment, the indigestible carbohydrate is present in the carbohydrate system in an amount of about 3.5% by weight. In another particularly desirable embodiment, the indigestible carbohydrate is present in the carbohydrate system in an amount of about 12% by weight.

In a particularly desirable embodiment, the carbohydrate system comprises about 68% by weight isomaltulose, about 8.0% by weight maltodextrin having a DE of 9 to 16, about 12% by weight fructooligosaccharides, about 7.0 % by weight Fibersol 2E insoluble dietary fiber, and a maximum of 5.0% by weight lactose.

In another particularly desirable embodiment, the carbohydrate system comprises about 68% by weight isomaltulose, about 8.0% by weight maltodextrin having a DE of 9 to 16, about 3.5% by weight fructooligosaccharides, about 15.5 % by weight Fibersol 2E insoluble dietary fiber, and a maximum of 5.0% by weight lactose.

### Macronutrients

The nutritional compositions including the carbohydrate systems as described herein may further comprise one or more optional additional macronutrients. The optional macronutrients include proteins, lipids, and carbohydrates in addition to the carbohydrate system described above, and combinations thereof. In some embodiments, the nutritional compositions are formulated as dietary products containing all three macronutrients for the pregnant or lactating woman.

Macronutrients suitable for use herein include any protein, lipid, or carbohydrate (in addition to the carbohydrate system) or source thereof that is known for or otherwise suitable for use in an oral nutritional composition, provided that the optional macronutrient is safe and effective for oral administration and is otherwise compatible with the other ingredients in the nutritional composition.

The concentration or amount of optional lipid, carbohydrate (including the carbohydrate system described herein), and protein in the nutritional compositions can vary considerably depending upon the particular product form (e.g., bars or other solid dosage forms, milk or soy-based liquids or other clear beverages, reconstitutable powders, etc.) and the various other formulations and targeted dietary needs. These optional macronutrients are most typically formulated within any of the embodied ranges described in the following table.

**Table 1. Exemplary amounts of carbohydrate, protein and lipid.**

| **Nutrient % Total Cal.** | **Embodiment A** | **Embodiment B** | **Embodiment C** |
|---|---|---|---|
| **Carbohydrate** | 0-98 | 2-96 | 10-75 |
| **Protein** | 0-98 | 2-96 | 5-70 |
| **Lipid** | 0-98 | 2-96 | 20-85 |

| | **Embodiment D** | **Embodiment E** | **Embodiment F** |
|---|---|---|---|
| **Carbohydrate** | 30-50 | 25-50 | 25-50 |
| **Protein** | 15-35 | 10-30 | 5-30 |
| **Lipid** | 35-55 | 1-20 | 2-20 |

| | | | |
|---|---|---|---|
| Each numerical value preceded by the term "about" | | | |

### Carbohydrate

Optional carbohydrates suitable for use in the nutritional compositions, in addition to the carbohydrate systems described herein, may be simple, complex, or variations or combinations thereof. Non-limiting examples of suitable carbohydrates include hydrolyzed or modified starch or cornstarch, maltodextrin, isomaltulose, sucromalt, glucose polymers, sucrose, corn syrup, corn syrup solids, rice-derived carbohydrate, glucose, fructose, lactose, high fructose corn syrup, honey, sugar alcohols (e.g., maltitol, erythritol, sorbitol), and combinations thereof.

Optional carbohydrates suitable for use herein also include soluble dietary fiber, non-limiting examples of which include gum Arabic, fructooligosaccharides (FOS), sodium carboxymethyl cellulose, guar gum, citrus pectin, low and high methoxy pectin, oat and barley glucans, carrageenan, psyllium, and combinations thereof. Insoluble dietary fiber is also suitable as a carbohydrate source herein, non-limiting examples of which include oat hull fiber, pea hull fiber, soy hull fiber, soy cotyledon fiber, sugar beet fiber, cellulose, corn bran, and combinations thereof.

### Protein

Optional proteins suitable for use in the nutritional compositions include hydrolyzed, partially hydrolyzed or non-hydrolyzed proteins or protein sources, and can be derived from any known or otherwise suitable source such as milk (e.g., casein, whey), animal (e.g., meat, fish, egg albumen), cereal (e.g., rice, corn), vegetable (e.g., soy, pea, potato), or combinations thereof. The proteins for use herein can also include, or be entirely or partially replaced by, free amino acids known for use in nutritional products, non-limiting examples of which include L-tryptophan, L-glutamine, L-tyrosine, L-methionine, L-cysteine, taurine, L-arginine, L-carnitine, and combinations thereof.

### Lipid

Optional lipids suitable for use in the nutritional composition include coconut oil, fractionated coconut oil, soy oil, corn oil, olive oil, safflower oil, high oleic safflower oil, high GLA-safflower oil, MCT oil (medium chain triglycerides), sunflower oil, high oleic sunflower oil, palm and palm kernel oils, palm olein, canola oil, flaxseed oil, borage oil, cottonseed oils, evening primrose oil, blackcurrant seed oil, transgenic oil sources, fungal oils, algae oils, marine oils (e.g., tuna, sardine), and so forth.

### Other Optional Ingredients

The nutritional compositions as described herein may further comprise other optional ingredients that may modify the physical, chemical, aesthetic or processing characteristics of the products or serve as pharmaceutical or additional nutritional components when used in the targeted population. Many such optional ingredients are known or otherwise suitable for use in medical food or other nutritional products or pharmaceutical dosage forms and may also be used in the compositions herein, provided that such optional ingredients are safe for oral administration and are compatible with the essential and other ingredients in the selected product form.

Non-limiting examples of such optional ingredients include preservatives, anti-oxidants, emulsifying agents, buffers, human milk oligosaccharides and other prebiotics, probiotics, nucleotides, carotenoids, pharmaceutical actives, additional nutrients as described herein, colorants, flavors, thickening agents and stabilizers, emulsifying agents, lubricants, and so forth, and combinations thereof.

A flowing agent or anti-caking agent may be included in the nutritional compositions as described herein to retard clumping or caking of the powder over time and to make a powder embodiment flow easily from its container. Any known flowing or anti-caking agents that are known or otherwise suitable for use in a nutritional powder or product form are suitable for use herein, non limiting examples of which include tricalcium phosphate, silicates, and combinations thereof. The concentration of the flowing agent or anti-caking agent in the nutritional product varies depending upon the product form, the other selected ingredients, the desired flow properties, and so forth, but most typically range from about 0.1% to about 4%, including from about 0.5% to about 2%, by weight of the composition.

A stabilizer may also be included in the nutritional compositions. Any stabilizer that is known or otherwise suitable for use in a nutritional product is also suitable for use herein, some non-limiting examples of which include gums such as xanthan gum. The stabilizer may represent from about 0.1% to about 5.0%, including from about 0.5% to about 3%, including from about 0.7% to about 1.5%, by weight of the nutritional composition.

The nutritional composition may further comprise any of a variety of vitamins, non-limiting examples of which include vitamin A, vitamin D, vitamin E, vitamin K, thiamine, riboflavin, pyridoxine, vitamin B12, niacin, folic acid, pantothenic acid, biotin, vitamin C, choline, inositol, salts and derivatives thereof, and combinations thereof.

The nutritional composition may also further comprise any of a variety of minerals known or otherwise suitable for use in nutritional compositions, non-limiting examples of which include phosphorus, magnesium, calcium as described hereinbefore, zinc, manganese, copper, iodine, sodium, potassium, chloride, selenium, and combinations thereof.

### Methods of Manufacture

The nutritional compositions for use in the nutrition systems of the present disclosure may be prepared by any known or otherwise effective manufacturing technique for preparing the selected product solid or liquid form. Many such techniques are known for any given product form such as nutritional liquids or powders and can easily be applied by one of ordinary skill in the art to the nutritional compositions described herein.

The nutritional compositions can therefore be prepared by any of a variety of known or otherwise effective formulation or manufacturing methods. In one suitable manufacturing process, for example, at least two separate slurries are prepared, that are later blended together, heat treated, standardized, and either terminally sterilized to form a retort composition or aseptically processed and filled to form an aseptic composition. Alternately, the slurries can be blended together, heat treated, standardized, heat treated a second time, evaporated to remove water, and spray dried to form a powder composition.

The slurries formed may include a carbohydrate-mineral (CHO-MIN) slurry and a protein-in-fat (PIF) slurry. Initially, the CHO-MIN slurry is formed by dissolving selected carbohydrates (e.g., carbohydrate system, etc.) in heated water with agitation, followed by the addition of minerals (e.g., potassium citrate, magnesium chloride, potassium chloride, sodium chloride, choline chloride, etc.). The resulting CHO-MIN slurry is held with continued heat and moderate agitation until it is later blended with the other prepared slurries.

The PIF slurry is formed by heating and mixing the oil (e.g., high oleic safflower oil, soybean oil, coconut oil, monoglycerides, etc.) and emulsifier (e.g., soy lecithin), and then adding oil soluble vitamins, mixed carotenoids, protein (e.g., whey protein, casein protein, etc.), carrageenan (if any), calcium carbonate or tricalcium phosphate (if any), and ARA oil and DHA oil (in some embodiments) with continued heat and agitation. The resulting PIF slurry is held with continued heat and moderate agitation until it is later blended with the other prepared slurries.

Water was heated and then combined with the CHO-MIN slurry, nonfat milk (if any), and the PIF slurry under adequate agitation. The pH of the resulting blend was adjusted to 6.6-7.0, and the blend was held under moderate heated agitation. ARA oil and DHA oil is added at this stage in some embodiments.

The composition is then subjected to high-temperature short-time (HTST) processing, during which the composition is heat treated, emulsified and homogenized, and then cooled. Water soluble vitamins and ascorbic acid are added, the pH is adjusted to the desired range if necessary, flavors (if any) are added, and water is added to achieve the desired total solid level. For aseptic compositions, the emulsion receives a second heat treatment through an aseptic processor, is cooled, and then aseptically packaged into suitable containers. For retort compositions, the emulsion is packaged into suitable containers and terminally sterilized. In some embodiments, the emulsions can be optionally further diluted, heat-treated, and packaged to form a desired ready-to-feed or concentrated liquid, or can be heat-treated and subsequently processed and packaged as a reconstitutable powder, e.g., spray dried, dry mixed, agglomerated.

The spray dried composition or dry-mixed composition may be prepared by any collection of known or otherwise effective techniques, suitable for making and formulating a nutritional powder. For example, when the powder composition is a spray-dried nutritional powder, the spray drying step may likewise include any spray drying technique that is known for or otherwise suitable for use in the production of nutritional powders. Many different spray drying methods and techniques are known for use in the nutrition field, all of which are suitable for use in the manufacture of the spray dried powder composition. Following drying, the finished powder may be packaged into suitable containers. Dryblending or drymixing may also be used to prepare the nutritional compositions of the present disclosure.

### Methods of Use

In some embodiments, the nutritional composition including the carbohydrate system as described above is administered to a pregnant woman to provide advantageous benefits to the woman during pregnancy and also to the offspring (baby) after delivery and later in life. In other embodiments, the nutritional composition including the carbohydrate system as described above is administered to the pregnant woman and additionally administered to the woman during lactation such that the suckling offspring and the mother continue to derive additional benefits as noted herein.

In one embodiment, the nutritional composition including the carbohydrate system as described herein is administered to the pregnant woman to blunt the glycemic response of digestible glucose polymers in the woman and to improve glycemia and insulinemia during the gestational period. In some embodiments, the pregnant woman may be diabetic and/or obese, and/or may have gestational diabetes mellitus, or may be at risk of being diabetic and/or obese or having gestational diabetes mellitus. As such, in some embodiments, the offspring of the mother may be at increased risk of obesity, glucose intolerance, and the like later in life.

In another embodiment, the nutritional composition including the carbohydrate system as described herein is administered to the pregnant woman and to the woman during lactation to blunt the glycemic response of digestible glucose polymers in the woman and to improve glycemia and insulinemia during the gestational and lactation periods. In some embodiments, the pregnant woman may be diabetic and/or obese, or may have gestational diabetes mellitus, or may be at risk of being diabetic and/or obese or having gestational diabetes mellitus. As such, in some embodiments, the offspring of the mother may be at increased risk of obesity, glucose intolerance, and the like later in life. Although generally less desirable, the nutritional composition including the carbohydrate system as described herein may be administered to the woman only during the lactation period and not during pregnancy.

In other embodiments, the nutritional composition including the carbohydrate system is administered to the pregnant woman, and optionally to the lactating woman, to prevent or reduce the incidence of long term adverse health effects in the offspring later in life. The methods of the present disclosure are effective for improving bone health in the offspring later in life.

In other embodiments, the nutritional composition including the carbohydrate system is administered to the pregnant woman, and optionally to the lactating woman, to prevent or reduce the incidence of long term adverse health effects in the offspring later in life. The methods of the present disclosure are effective for improving cognitive and CNS development in offspring later in life, including, for example, improved learning and cognitive outcomes.

As noted herein, the nutritional composition including the carbohydrate system described herein may be administered to a pregnant woman, or may be administered to a pregnant woman and then further to the woman during lactation. When the nutritional composition including the carbohydrate system described herein is administered to a pregnant woman, it is generally administered for a period of at least 1 month, including at least 2 months, including at least 3 months, including at least 4 months, including at least 5 months, including at least 6 months, including at least 7 months, including at least 8 months, and including substantially during the entire pregnancy. In a desirable embodiment, the nutritional composition including the carbohydrate system described herein is administered in a continuous, day to day manner, although administration in a manner other than day to day or every day is within the scope of the methods of the present disclosure. When the nutritional composition of the present disclosure including the carbohydrate system as described herein is administered to a lactating woman, it may be administered for the entire period of lactation, or for a lesser period of time, although it is generally desirable to administer the nutritional composition for the entire period of lactation. In a desirable embodiment, the nutritional composition including the carbohydrate system described herein is administered during lactation in a continuous, day to day manner, although administration in a manner other than day to day or every day is within the scope of the methods of the present disclosure.

The carbohydrate system as described herein for administration to the pregnant woman may be administered to the woman such that the daily intake is from about 20 grams to about 175 grams, including from about 50 grams to about 175 grams, including from about 75 grams to about 150 grams, including from about 75 grams to about 125 grams, including from about 90 grams to about 125 grams, and further including from about 100 grams to about 125 grams. When the carbohydrate system is administered to the lactating woman, it may be administered such that the daily intake is from about 20 grams to about 210 grams, including from about 50 grams to about 210 grams, including from about 75 grams to about 210 grams, including from about 100 grams to about 210 grams, including from about 125 grams to about 200 grams, and further including from about 150 grams to about 175 grams.

### EXAMPLES

The following examples illustrate specific embodiments and/or features of the nutritional compositions and methods of the present disclosure. The examples are given solely for the purpose of illustration and are not to be construed as limitations of the present disclosure, as many variations thereof are possible without departing from the spirit and scope of the disclosure. All exemplified amounts are weight percentages based upon the total weight of the composition, unless otherwise specified.

### Example 1. Effect of CHO Systems on Cognitive Development

In this Example, a well-established rat model of developmental programming (the "maternal over-nutrition" model) was used to evaluate the effect of maternal nutritional intervention, using various carbohydrate systems with different glycemic indices (GI), during gestation on the offspring glycemic control as a nutritional strategy to improve cognition later in life in the offspring.

*Animal maintenance and experimental procedures.* Female Sprague-Dawley virgin rats (10-wk-old) and male Sprague-Dawley rats (13-wk-old) were obtained from Charles River Laboratories (Orleans Cedex, France). Protocols for all experimental procedures were conducted in accordance with the ethical guidelines for animal experimentation at the Spanish National Research Council (RD 1201/2005 October 10). An obesogenic (20% fat, 24% protein, 49% CHO) diet was used to feed the female rats for 6 weeks before mating. This high fat diet is rich in sucrose (38% total CHO), maltodextrins (22% total CHO), and corn starch (30% total CHO), hereafter it will be called high GI group (GI=447). Males were kept on a separate room until mating, feeding AIN93M diet. After mating, they were euthanized. During gestation, the pregnant rats were divided into two groups: one was kept upon the high GI diet, while the other was fed a high fat diet but containing mainly isomaltulose (68% total CHO) and Fibersol (15.5% total CHO), hereafter named low GI group. Besides, a control group of female rats was kept on standard AIN93M diet (4% fat, 13% protein, 75% CHO) before mating and fed AIN93G diet (7% fat, 18% protein, 67% CHO) during gestation. All diets are listed on Table 2.

Once deliveries took place, all dams were fed the same AIN93G diet until weaning at PND21. Within 24h after delivery, each dam was given an eight-pup litter, 5 males and 3 females. After weaning the dams were euthanized and the male pups were weaned onto standard AIN93G and housed in groups of 4 animals per cage until 6 week of age. During the growing and testing periods, offspring rats were housed individually with free access to the control standard AIN93M diet.

**Table 2. Total theoretical daily glycemic load (GL) was calculated by first multiplying the amount of each carbohydrate contained in a daily dietary intake by its glycemic index (with the use of glucose as the reference food), then by summing the values from all CHO sources. Daily dietary glycemic load thus represents the quality and quantity of carbohydrate intake and the interaction between the two.**

| | **Standard control rodent diets** | | **Experimental obesogenic rodent diets** | |
|---|---|---|---|---|
| | **AIN93G** | **AIN93M** | **HF Low GI** | **HF** |
| **Total Protein** | 18.30 | 12.89 | 24.19 | 24.19 |
| Calcium caseinate | 97.60 | 98.41 | 97.65 | 97.81 |
| **Total Fat** | 7.00 | 4.10 | 20.50 | 20.50 |
| Lard Fat | 0.00 | 0.00 | 98.46 | 99.99 |
| **Total HC** | 64.59 | 71.19 | 49.11 | 49.42 |
| Maltodextrin (GI=95) | 18.79 | 20.53 | 7.76 | 24.43 |
| Lactose (GI=46) | -- | -- | 5.15 | -- |
| Isomaltulose (GI=32) | -- | -- | 67.88 | -- |
| Sucrose (GI=65) | 14.37 | 13.91 | 0.33 | 36.07 |
| Glucose (GI=100) | -- | -- | 0.08 | -- |
| Cornstarch (GI=85) | 59.48 | 58.54 | -- | 31.60 |
| Fibersol 2E (GI=5) | -- | -- | 15.47 | -- |
| FOS (GI=0) | -- | -- | 3.49 | -- |
| Cellulose (GI=0) | 7.18 | 7.02 | -- | 7.90 |
| **Glycemic Load (GL)** | 910 | 927 | 256 | 625 |

*Behavioral Tests.* Behavioral tests were performed from PND169 (water maze) and PND200 (object recognition). In order to assess learning and memory abilities, two main paradigms were assayed: Morris water maze and novel object recognition. The water maze was performed by using a 1.5m diameter x50 cm height pool, filled up to 30 cm with warm water (23±1°C). A 12cm diameter platform was hidden 1 cm below the water level. The pool was surrounded by an opaque curtain to isolate it from the testing room. Two white rectangles, one with a vertical black strip and the other with two horizontal black strips, were placed one in front of the other to be used as landmarks. All the animals performed 4 daily trials, with an inter-trial interval of 10 min. The platform and the entrance point to the pool randomly changed on every trial, so the platform was located once in each of the four quadrants (NE, SE, NW, SW) every training day and each animal enter the pool once from each geographic point every day. The landmarks were moved as well in order to keep them correctly aligned with the platform. Rats were allowed to swim for 1 min or until they reached the platform. If they failed to find it, experimenter guided them to the platform. In both cases, the animal remained 30s on top of the platform in order to let it learn how to reach the escape platform. Training lasted until the learning curve reached the asymptote (14d). Then, 24h after the last training, the rats were placed into the pool once again, in the absence of the platform, to test their memory. If the animal had learned the relationship between the platform and the landmarks, it would spend a longer time searching for it in the target quadrant (the one where the platform should be) than in the other three quadrants. Regarding the novel object recognition, a four arena box was used, each arena measuring 45x45x45 cm. During three days, rats were put into the arena for 20 min in order to get them habituated to the testing scenario. On the fourth day, rats were presented two objects and let them observe the objects for 10 min. On the next day, one of these familiar objects was substituted by a new one (novel object) and the rat was allowed to explore both objects for 5 min. If the animal remembered which object was already there on the previous day, it should be attracted to the novel object and spent a longer time observing it, due to their natural neophilia. Objects were previously tested on naïve rats to assure they have the same preference for all of them. All trials were recorded and analyzed using Viewer3 software (Biobserve, Germany). Data were plotted on Microsoft Excel. Statistical analyses were performed with Statgraphs software (San Diego, CA). Significant differences were set at p<0.05.

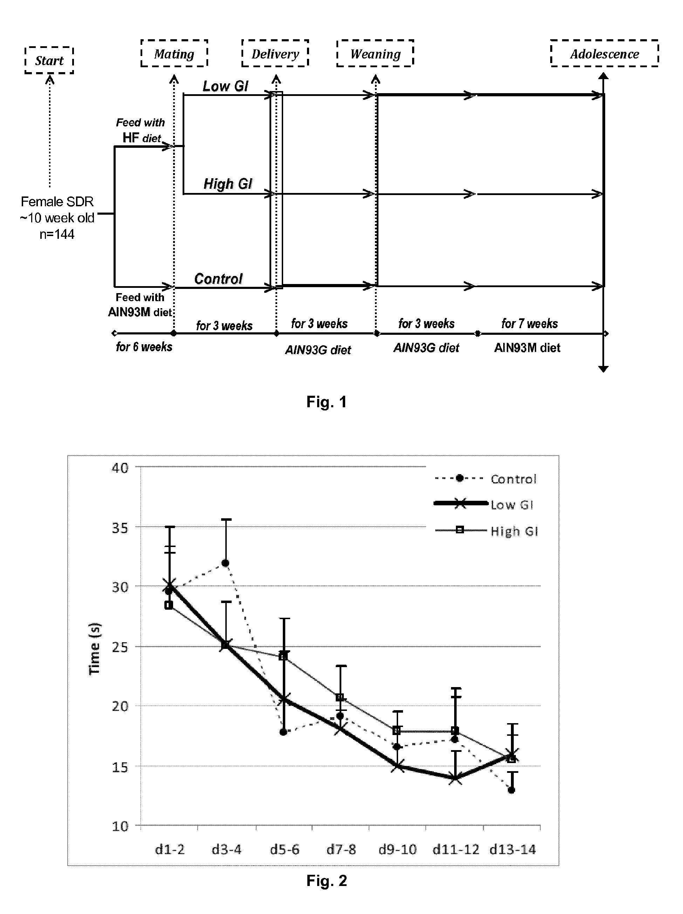

*Results.* Data from the water maze training are plotted on Figure 1. To simplify nomenclature, we hereafter refer to the offspring of mothers fed low GI diet during gestation as Low GI rats, and High GI rats will be the offspring of mothers fed high GI diet during lactation. The 2-way ANOVA raised no statistical differences amongst the three learning curves. However, the comparison between Low GI animals and High GI animals shows that the former ones are able to learn where the platform is quicker than the latter ones, while the control group exhibited the most pronounced slope between block 2 and 3. No differences in swim speed were found; therefore differences are due to shorter swim tracks. By the 7th block, all animals seemed to have learnt how to find the platform.

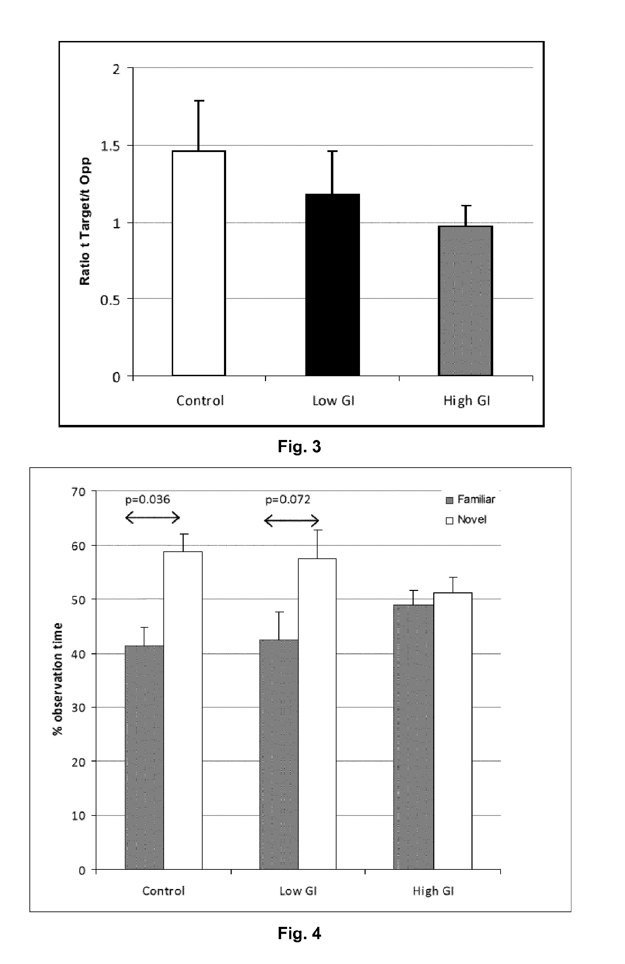

Results from the test session are shown in Figure 3. One way ANOVA raised no statistically significant differences among the three groups. However, control rats spent a longer time in the target quadrant (where the platform should have been) than in the opposite one, clearly showing that they were able to remember the relationship between the landmarks and the platform. In contrast, High GI rats were not able to remember this relationship and they spent the same time searching for the platform in the right than in the wrong quadrants. The Low GI rats seemed to remember the right quadrant, since they showed a trend to spend a longer time searching for the platform in the target, although their performances were not as good as those of the control/normal rats.

Data from novel object recognition are plotted in Figure 4. In this task, total observation time was split into time spent exploring the familiar object and time spent exploring the new one. A t-test was applied to find differences between familiar and novel observation times for each group. High GI rats spent half the time exploring each object, denoting they did not remember which one of them had been already presented previously. Again, normal control rats recognized the novel object and spent a significantly longer time exploring this new object. Low GI rats also recognized novelty, since they explored the novel object for longer time than the familiar one, although statistical significance was not reached (p=0.07).

*Conclusions.* The above-described experiments lead to the conclusion that low GI diets fed to mothers during gestation improve learning abilities of rats during the water maze training phase when compared to the high GI diet offspring. Low GI rats were better able to recall the landmarks and clues to locate the platform quadrant during the memory test, 24 hours after the training, and better remembered the already explored object in the novel object recognition paradigm and spent more time exploring the novel object. In summary, progeny from mothers fed an obesogenic high GI diet before and during pregnancy seem to have certain impairments in cognitive abilities related to learning and memory. The use of a low GI diet during gestation could counteract some of these deficits.

### Example 2. Effect of CHO Systems on Bone Development

In this Example, a well-established rat model of developmental programming (the "maternal over-nutrition" model) was used to evaluate the effect of maternal nutritional intervention, using various carbohydrate systems with different glycemic indices (GI), during gestation on the offspring glycemic control as a nutritional strategy to improve bone health later in life in the offspring.

*Animal maintenance and experimental procedures.* Female Sprague-Dawley virgin rats (10-wk-old) were obtained from Charles River Laboratories (Orleans Cedex, France). Protocols for all experimental procedures were conducted in accordance with the ethical guidelines for animal experimentation at the Spanish National Research Council (RD 1201/2005 October 10). *Rats were housed* individually with free access either to the control standard AIN 93M (American Institute of Nutrition) diet or the highly palatable obesogenic lard diet (HF). The AIN 93M diet contained 4% fat, 12.9% protein, 70% carbohydrates and 5% fiber, while the HF diet consisted of 20.5% fat, 24.2% protein, 41.5% carbohydrates and 7.9% fiber. After 6 weeks of eating these diets, the rats were bred with 13 week old male AIN 93M-fed Sprague Dawley rats. After mating, and only during gestation period (≈21 days), the dams fed with HF diet were then randomly assigned to one of two experimental obesogenic HF-diets, containing different types of carbohydrates with different GI, (Table 3 set forth below).

**Table 3. Total daily glycemic load (GL) was calculated by first multiplying the amount of each carbohydrate contained in a daily dietary intake by its glycemic index (with the use of glucose as the reference food) then by summing the values from all CHO sources. Daily dietary glycemic load thus represents the quality and quantity of carbohydrate intake and the interaction between the two.**

| | Standard control and obesogenic rodent diets | | | Experimental obesogenic rodent diets | |
|---|---|---|---|---|---|
| | AIN93G | AIN93M | HF | HF Low GI | HF High GI |
| Total Protein | 18.30 | 12.89 | 24.19 | 24.19 | 24.19 |
| Calcium caseinate | 97.60 | 98.41 | 97.81 | 97.65 | 97.81 |
| Total Fat | 7.00 | 4.10 | 20.50 | 20.50 | 20.50 |
| Lard fat | 0.00 | 0.00 | 99.99 | 98.46 | 99.99 |
| Total HC | 64.59 | 71.19 | 49.42 | 49.11 | 47.54 |
| Maltodextrin (GI=95) | 18.97 | 20.53 | 24.43 | 7.76 | 82.68 |
| Lactose (GI=46) | -- | -- | -- | 5.15 | 5.16 |
| Isomaltulose (GI=32) | -- | -- | -- | 67.88 | -- |
| Sucrose (GI=65) | 14.37 | 13.91 | 36.07 | 0.33 | 1.70 |
| Glucose (GI=100) | -- | -- | -- | 0.08 | 0.85 |
| Cornstarch (GI=85) | 59.48 | 58.54 | 31.60 | -- | -- |
| Fibersol 2E (GI=5) | -- | -- | -- | 15.47 | -- |
| FOS (GI=0) | -- | -- | -- | 3.49 | -- |
| Cellulose (GI=0) | 7.18 | 7.02 | 7.90 | -- | 9.60 |
| Glycemic Load (GL) | 910 | 927 | 625 | 256 | 756 |

Meanwhile, the dams fed with AIN 93M diet continued on AIN 93G diet, (consisted of 7% fat, 18.3% protein, 57.4% carbohydrates and 7.2% fiber), during the gestation period (control group). Body weight was monitored at least weekly and food intake was measured 3 times per week by weighing the diet in the feed containers. After delivery and throughout the lactation period (≈21 days), regardless of the diets consumed during pre- and pregnancy periods, all dams were fed with the control standard AIN 93G diet. Within 24 hours of birth, litter size was adjusted to 8 male pups per litter. After weaning, the dams were euthanized and the male pups were weaned onto standard AIN 93G and multi-housed in cages until 6 weeks of age. During the growing period until the adolescence (90-days-old), offspring rats were housed individually with free access to the control standard AIN 93M diet.

*Bone architecture analysis.* Micro-computed tomography (micro-CT) is an emerging technique for the non-destructive assessment and analysis of the three-dimensional trabecular bone structure. Micro-CT is one of the approaches to image and quantify cancellous bone in three dimensions. The development of micro-CT was first driven by the need for having a highly precise and accurate means of reconstructing the complex architecture of bone tissue at a high resolution and is becoming a crucial parameter in evaluating bone growth and disease pathogenesis. Scanning with micro-CT could be achieved at resolutions as low as 5 µm, allowing the determination of porosities and subtle modelling and remodelling events of the bone tissue. Furthermore, true three dimensional image reconstructions permit the assessment of bone microarchitecture as a three dimensional structure, providing critical information to images collected through the histomorphometry. An overall characterization of both bone quantity and quality will certainly lead to a more accurate determination of the interdependence of these factors in defining resilience and tendency to fracture. In the present study femoral cancellous bones were assessed using MicroCT-40 computed tomography system (Scanco Medical, Basserdorf, Switzerland), and were performed by the same technologist. Micro-CT imaging was coupled with stereological methods to estimate bone volume fraction (BV/TV, is a 3-D parameter that related bone volume per unit of volume and is one of the most important parameter to characterized cancellous bone architectural morphology) and 3-D parameters of trabecular architecture including: trabecular thickness (Tb.Th in µm, average thickness of trabeculae), trabecular separation (Tb.Sp in µm, the average distance between trabeculae, representing the amount of marrow space), and structure model index (SMI, is a parameter used to quantify the trabecular network indicating the prevalence of the rod-like (level 3) or plate-like (level 0) structures of the trabecular architecture. The new forming bone is constituted by high number more rod-like plates, while in a consolidate bone is constituted by plate-like way)

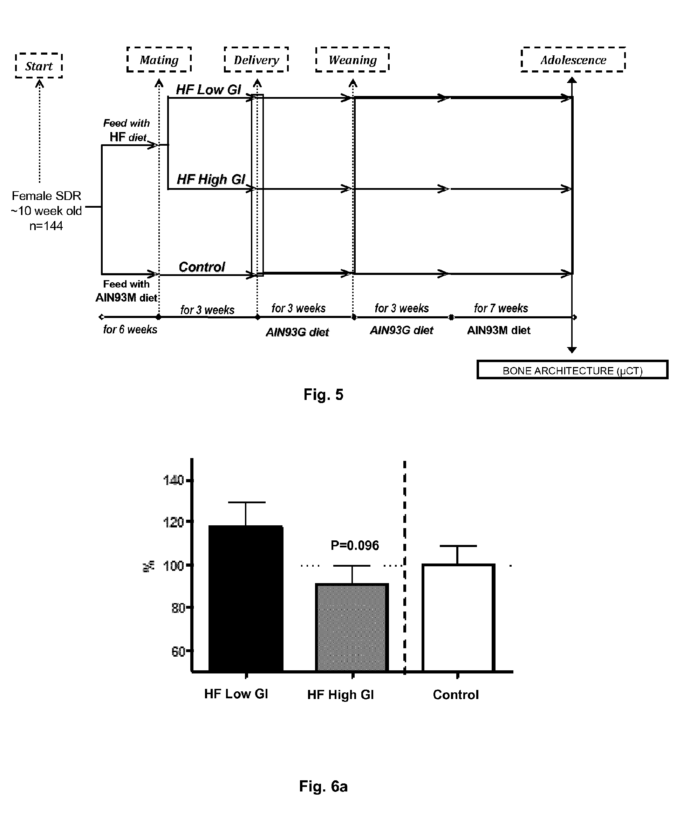

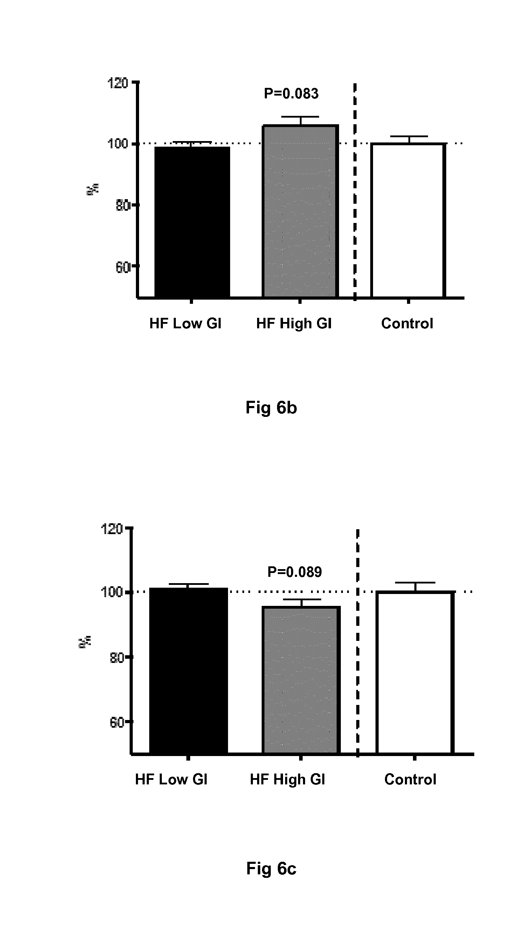

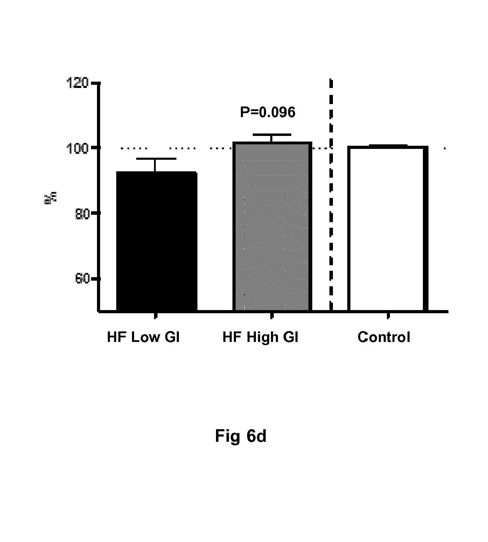

*Results.* Micro-CT analysis of bone showed that, in the gestating rats, nutritional intervention with a high fat/high GI diet induced alterations in the trabecular architecture of femur in the mail offspring. This alteration of the overall microstructure of the bones implies that the femur of offspring of High fat/High GI diet group may have a lower resistance against bone breakage and collapsing in comparison to the control group. However, nutritional intervention with a High fat/low GI diet (containing the dietary carbohydrate systems described herein), not only preserved the alterations in trabecular architecture induced by the maternal High fat diet, but also exerted an improvement in the architecture of trabecular bone at femurs in comparison to control group (Figure 6).

*Conclusions.* A low GI diet containing the mixture described herein during gestation may exert a protective effect against the structural bone alterations induced by the high fat diet (in insulin resistance conditions) in their offspring. The positive effect promoted by the low GI diet during gestation may maximize the peak of bone mass during the healthy growing period until adolescence in offspring in healthy conditions as a strategy to increase bone strength and to prevent bone fragility later in life.

## Claims

1. A method of improving a characteristic in an offspring, said characteristic selected from bone health or cognition, comprising administering a nutritional composition comprising a carbohydrate system, the carbohydrate system comprising a slow rate of digestion simple carbohydrate, a complex carbohydrate, a nonabsorbent carbohydrate, and/or an indigestible oligosaccharide to a woman during the gestational and/or lactation period of said offspring.

2. The method according to claim 1, wherein said characteristic is bone health.

3. The method of according to either claim 1 or claim 2, wherein said characteristic is cognition.

4. The method according to any preceding claim, wherein said improvement in cognition results in improved learning in said offspring.

5. The method according to any preceding claim, wherein said woman has a condition selected from overweight, obesity, diabetes, gestational diabetes mellitus, and a combination thereof.

6. The method according to any preceding claim, wherein the slow rate of digestion simple carbohydrate is selected from the group consisting of isomaltulose, sucromalt, and combinations thereof.

7. The method according to any preceding claim, wherein the complex carbohydrate is selected from the group consisting of a maltodextrin, corn starch, rice starch, wheat starch, and combinations thereof.

8. The method according to any preceding claim, wherein the complex carbohydrate comprises maltodextrin having a Dextrose Equivalent of from 5 to 25, preferably from 9 to 16.

9. The method according to any preceding claim, wherein the nonabsorbent carbohydrate is selected from the group consisting of inulin, dietary insoluble fibers, digestion resistant maltodextrins, and combinations thereof.

10. The method according to any preceding claim, wherein the indigestible oligosaccharide is selected from the group consisting of fructooligosaccharides, galactooligosaccharides, trans-galactooligosaccharides, xylooligosaccharides, and combinations thereof.

11. The method according to any preceding claim, wherein the nutritional composition is administered daily for at least the last three months of the pregnancy, preferably at least the last six months of pregnancy.

12. The method according to any preceding claim, wherein the woman consumes from 20 to 175 grams of the carbohydrate system per day.

13. The method according to any preceding claim, wherein the carbohydrate system is comprised of from 40% to 80% by weight slow rate of digestion simple carbohydrate, from 1% to 15% by weight complex carbohydrate, from 5% to 25% by weight nonabsorbent carbohydrate, and from 1% to 20% by weight indigestible carbohydrate.

14. The method according to any preceding claim, wherein the carbohydrate system is comprised of from 60% to 70% by weight slow rate of digestion simple carbohydrate, from 6% to 10% by weight complex carbohydrate, from 5% to 20% by weight nonabsorbent carbohydrate, and from 2% to 15% by weight indigestible carbohydrate.

15. The method according to claim 1, wherein the nutritional composition is administered to the woman during the gestational period of said offspring, and wherein said woman has a condition selected from overweight, obesity, diabetes, and combinations thereof.
